# EUROPEAN PATENT APPLICATION

(11) **EP 4 559 467 A1**
(43) Date of publication of application: **28.05.2025**
(21) Application number: 23842283.6
(22) Date of filing: 18.07.2023
(51) Int. Cl.: A61K 31/704, A61K 31/522, C07D 473/02, A61P 35/00, A61K 9/127

(54) **USE OF IMIDAZOLINONE DERIVATIVE COMBINED WITH DOXORUBICIN IN TREATMENT OF TUMORS**

(30) Priority: 20.07.2022 CN 202210851902
(71) Applicant: Kangbaida (Sichuan) Biotechnology Co., Ltd., Chengdu City, Sichuan Province (CN)
(72) Inventor: YE, Fei, Chengdu, Sichuan 610000 (CN); WEI, Yonggang, Chengdu, Sichuan 610000 (CN); XU, Xuezhen, Chengdu, Sichuan 610000 (CN); LIU, Bing, Chengdu, Sichuan 610000 (CN); SU, Guizhuan, Chengdu, Sichuan 610000 (CN); GAO, Zhiwen, Chengdu, Sichuan 610000 (CN); SUN, Yi, Chengdu, Sichuan 610000 (CN)
(74) Representative: Carpmaels & Ransford LLP
(86) International application number: PCT/CN2023/107814
(87) International publication number: WO 2024/017220

(57) **Abstract**

Provided is a use of a compound represented by general formula (I) in combination with doxorubicin in the preparation of an anti-cancer drug

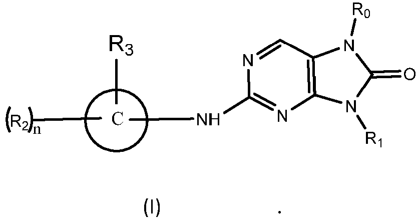

## Description

### TECHNICAL FIELD

The present invention relates to the use of an imidazolinone derivative for the treatment of a tumor in combination with Doxorubicin.

### BACKGROUND ART

Doxorubicin is a broad-spectrum anti-tumor drug, and one of its main mechanisms for treating cancer is directly destroying the DNA of cancer cells, causing massive DNA damage and ultimately killing the cancer cells. DNA damage response repair (DDR) is a self-protective mechanism that is initiated when cells experience DNA damage, which may lead to cell cycle arrest and the repair of damaged DNA, thereby reducing the sensitivity of cancer cells to Doxorubicin. In clinical practice, this reduced sensitivity is seen as a decrease in the therapeutic effect of Doxorubicin.

DNA double-strand break (DSB) is a highly harmful form of DNA damage in cells, and DNA double-strand breaks that are not repaired in time are closely associated with the canceration of cells. Non-homologous end-joining (NHEJ) is one of the main pathways for the repair of DNA double-strand breaks in cells. In NHEJ, the DSB end is first recognized and bound by Ku70/80 and then binds to a DNA-dependent protein kinase catalytic subunit (DNA-PKcs) to form a DNA-dependent protein kinase (DNAPK), i.e., an NHEJ initiation complex (DNAPK). Subsequently, two DNAPKs bind to the ends of damaged DNA while recruiting subsequent NHEJ repair factors (XRCC4 and XLF) and DNA ligase IV (LigIV) to repair the damaged DNA.

WO 2021209055 discloses the use of an imidazolinone derivative in the preparation of a drug for use in the treatment of a cancer, in which the compounds described in the specification have high selectivity and significant inhibitory activity against DNA-PK.

### SUMMARY OF THE INVENTION

An objective of the present invention is to provide the use of an imidazolinone derivative for the treatment of a tumor in combination with Doxorubicin, so as to overcome the deficiencies of the prior art.

In one or more embodiments of the present application, there is provided the use of a compound of general formula (I), or a stereoisomer, solvate, prodrug, metabolite, pharmaceutically acceptable salt or co-crystal thereof as an active ingredient for the treatment of a tumor in combination with Doxorubicin: 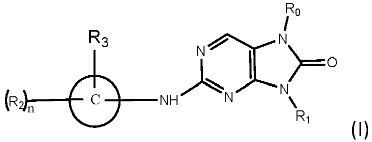 wherein 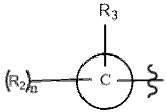 is 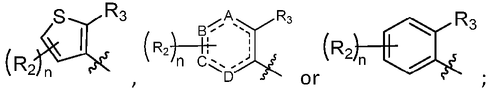
-̅ -̅ -̅ is a single bond or double bond; in 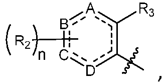 A, B, C, D are each independently C or N, and at least one of A, B, C and D is N;
R₀ is H, C₁₋₆ alkyl or cyclopropyl, wherein the C₁₋₆ alkyl is optionally further substituted with one or more substituents selected from halogen and deuterium;
R₁ is 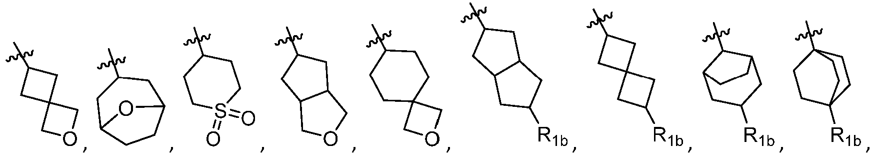 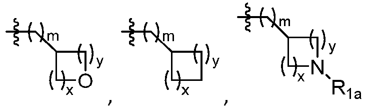 or pyridyl, and R₁ is optionally further substituted with 1 or 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R₂ is H, cyano, =O, carboxyl, -C(=O)NR₂ₐR_{2b}, C₁₋₆ alkoxy, C₁₋₆ alkyl, halogen, -S(=O)₂R₂ₐ or -C(=O)OC₁₋₆ alkyl, wherein the C₁₋₆ alkyl, -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1, 2 or 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 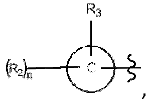 R₀, R₂, and R₃ simultaneously satisfy the following conditions, R₁ is not 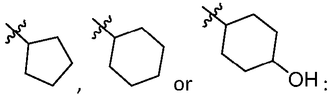 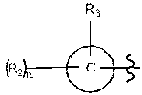 is 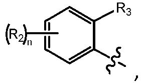 n is 1, R₀ is H or methyl, R₂ is methoxy or -S(=O)₂Me, and R₃ is methyl.

In one or more embodiments of the present application, R₁ is 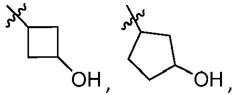 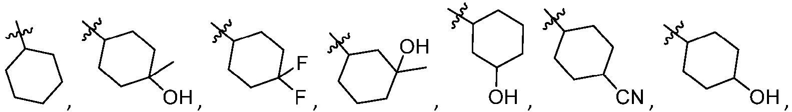 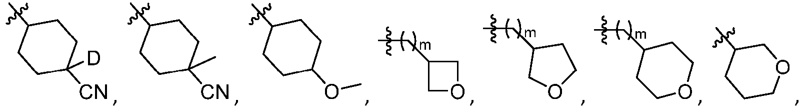 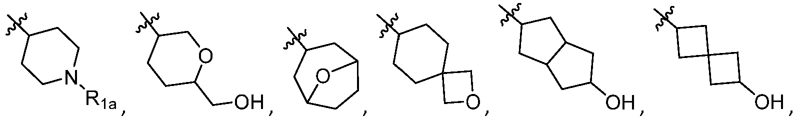 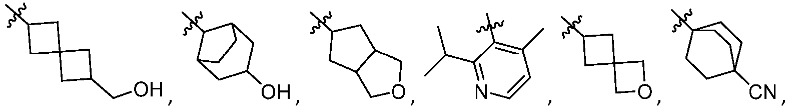 or 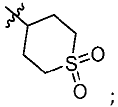
R₁ₐ is H or C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 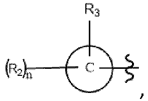 R₀, R₂, and R₃ simultaneously satisfy the following conditions, R₁ is not 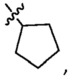 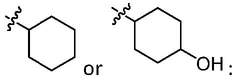 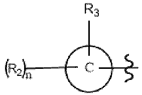 is 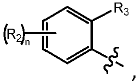 n is 1, R₀ is H or methyl, R₂ is methoxy or -S(=O)₂Me, and R₃ is methyl.

In one or more embodiments of the present application, 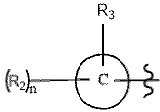 is 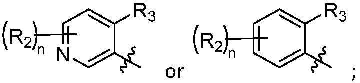
R₀ is H, C₁₋₄ alkyl or cyclopropyl, wherein the C₁₋₄ alkyl is optionally further substituted with one or more substituents selected from halogen and D;
R₁ is 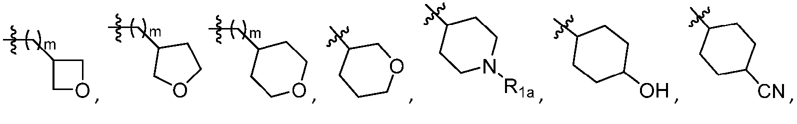 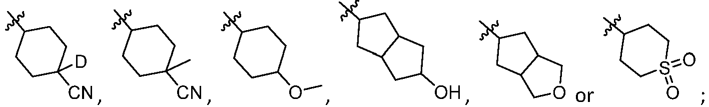
R₁ₐ is H, C₁₋₆ alkyl or -C(=O)C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D and halogen;
alternatively, R₂ₐ and R_{2b} together with the atoms to which they are attached form a 5- to 6-membered heterocyclyl, which contains 1 to 3 heteroatoms selected from N, O and S and is also optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 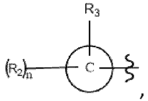 R₀, R₂, and R₃ simultaneously satisfy the following conditions, R₁ is not 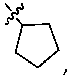 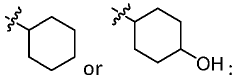
when 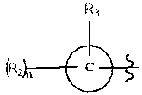 is 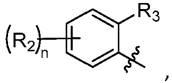 n is 1, R₀ is H or methyl, R₂ is methoxy or -S(=O)₂Me, and R₃ is methyl.

In one or more embodiments of the present application, there is provided the use of a compound of general formula (II), or a stereoisomer, solvate, prodrug, metabolite, pharmaceutically acceptable salt or co-crystal thereof as an active ingredient for the treatment of a tumor in combination with Doxorubicin: 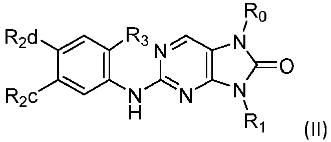 wherein
R₀ is H, C₁₋₆ alkyl or cyclopropyl, wherein the C₁₋₆ alkyl is optionally further substituted with one or more substituents selected from halogen and deuterium;
R₁ is 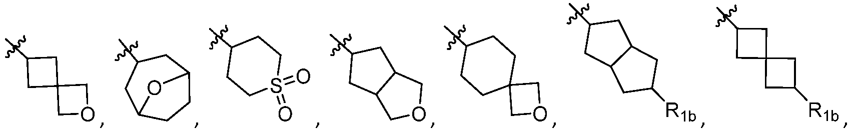 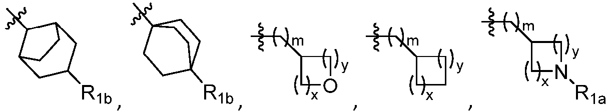 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R_{2c} is H, cyano, halogen or C₁₋₆ alkoxy;
R_{2d} is H, cyano, carboxyl, -C(=O)NR₂ₐR_{2b}, C₁₋₆ alkyl, halogen, -S(=O)₂R₂ₐ or -C(=O)OC₁₋₆ alkyl, wherein the C₁₋₆ alkyl and -C(=O)OC₁₋₆ alkyl is optionally substituted with one or more substituents selected from halogen and deuterium;
R₂ₐ and R_{2b} are H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 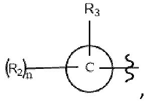 R₀, R₂, and R₃ simultaneously satisfy the following conditions, R₁ is not 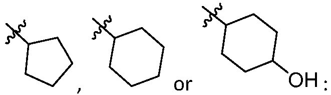 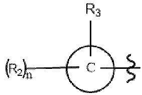 is selected from 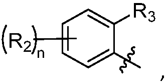 n is 1, R₀ is H or methyl, R₂ is methoxy or - S(=O)₂Me, and R₃ is methyl.

In one or more embodiments of the present application, there is provided the use of a compound of general formula (III), or a stereoisomer, solvate, prodrug, metabolite, pharmaceutically acceptable salt or co-crystal thereof as an active ingredient for the treatment of a tumor in combination with Doxorubicin: 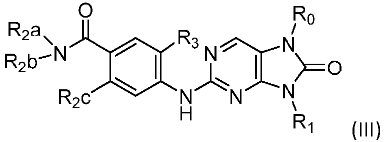 wherein
R₀ is H, C₁₋₆ alkyl or cyclopropyl, wherein the C₁₋₆ alkyl is optionally further substituted with one or more substituents selected from halogen and deuterium;
R₁ is 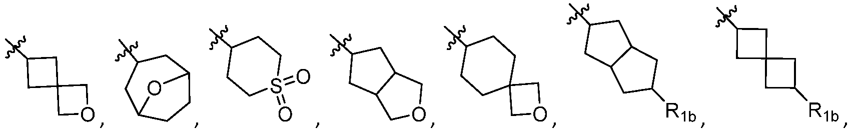 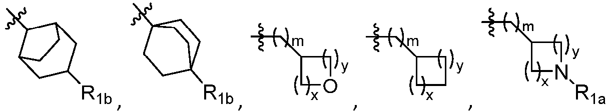 or pyridyl, and R₁ is optionally further substituted with 1 to 2 substituents selected from D, halogen, cyano, hydroxyl, C₁₋₆ alkyl and C₁₋₆ alkoxy;
R_{1b} is H, OH, cyano, or hydroxyl substituted C₁₋₆ alkyl;
R₂ₐ and R_{2b} are each independently H, C₁₋₆ alkyl, or 3- to 5-membered cycloalkyl, wherein the C₁₋₆ alkyl is optionally further substituted with one or more substituents selected from D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R_{2c} is H, cyano, halogen or C₁₋₆ alkoxy, wherein the C₁₋₆ alkoxy is optionally substituted with one or more deuterium;
R₃ is halogen or C₁₋₆ alkyl, wherein the C₁₋₆ alkyl is optionally further substituted with 1 to 3 substituents selected from D or halogen;
m is 0 or 1;
n is 0, 1 or 2; and
x and y are each independently 1, 2 or 3.

In one or more embodiments of the present application, there is provided the use of a compound of general formula (IV), or a stereoisomer, solvate, prodrug, metabolite, pharmaceutically acceptable salt or co-crystal thereof as an active ingredient for the treatment of a tumor in combination with Doxorubicin: 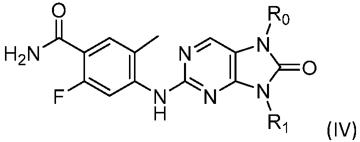 wherein
R₀ is H, C₁₋₆ alkyl or cyclopropyl, wherein the C₁₋₆ alkyl is optionally further substituted with one or more substituents selected from halogen and deuterium; and
R₁ is -(CH)ₘ-4- to 7-membered carbocyclyl, -(CH)ₘ-4- to 7-membered heterocyclyl, -(CH)ₘ-8- to 12-membered bridged ring, -(CH)ₘ-7- to 12-membered spiro ring, wherein the -(CH)ₘ-4- to 7-membered carbocyclyl, -(CH)ₘ-4- to 7-membered heterocyclyl, -(CH)ₘ-8- to 12-membered bridged ring, or -(CH)ₘ-7- to 12-membered spiro ring is optionally further substituted with one or more substituents selected from hydroxy, cyano, halogen, =O, C₁₋₆ alkyl, C₁₋₆ alkoxy, and hydroxy substituted C₁₋₆ alkyl.

In one or more embodiments of the present application,
R₀ is H, C₁₋₆ alkyl or cyclopropyl, wherein the C₁₋₆ alkyl is optionally further substituted with one or more substituents selected from halogen and deuterium;
R₁ is 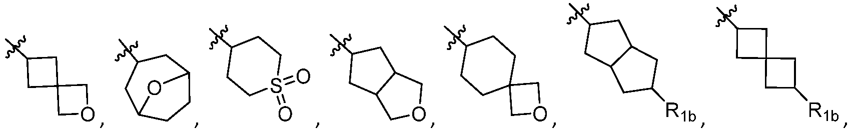 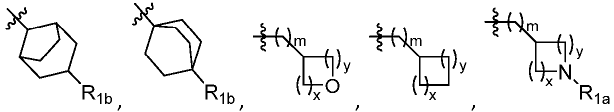 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
m is 0 or 1; and
x and y are each independently 1, 2 or 3.

In one or more embodiments of the present application,
R₀ is C₁₋₄ alkyl, wherein the C₁₋₄ alkyl is optionally further substituted with one or more substituents selected from halogen and deuterium; and
R₁ is

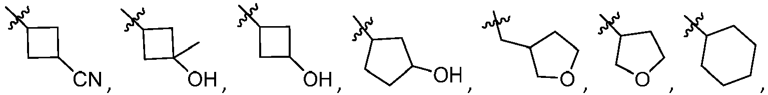

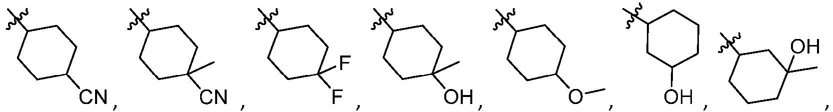

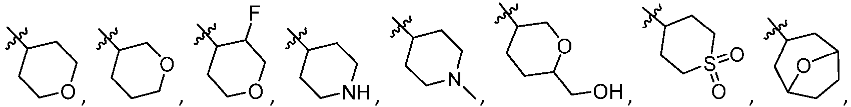

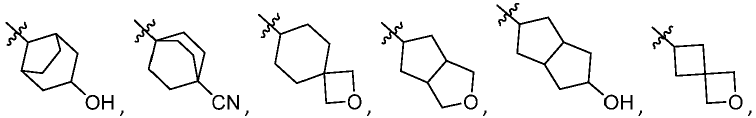

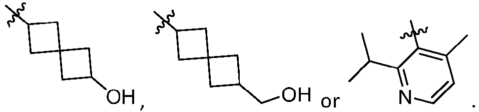

In one or more embodiments of the present application, the active ingredient is selected from:

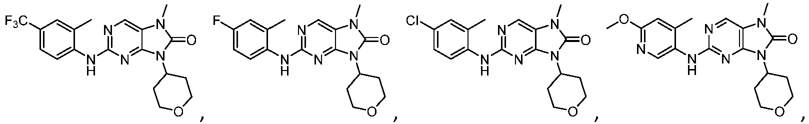

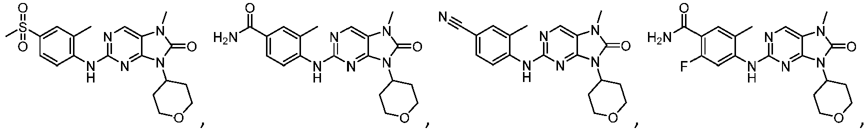

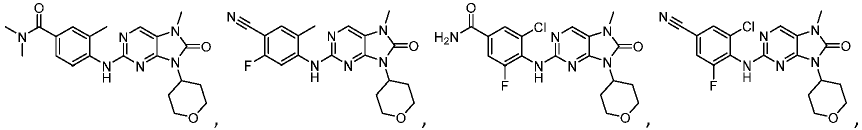

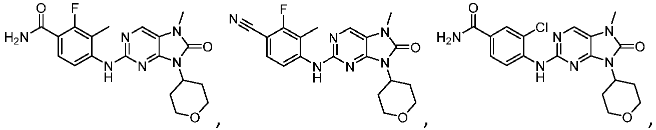

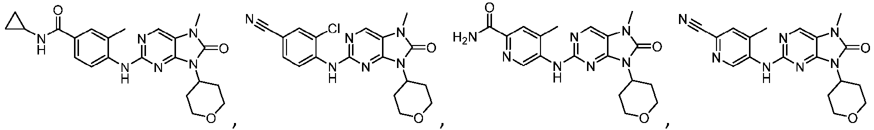

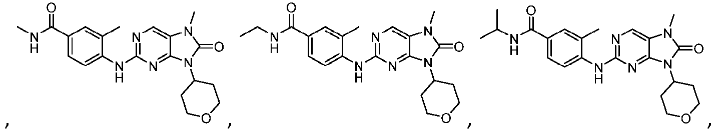

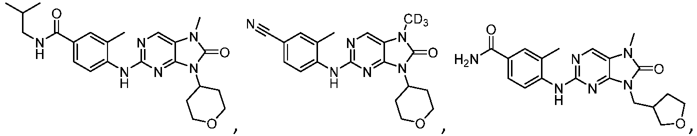

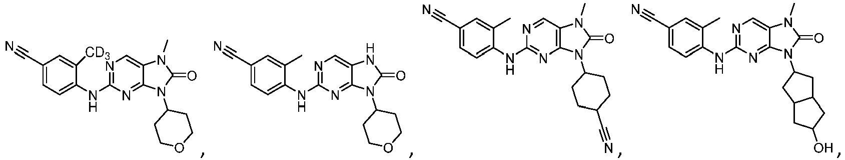

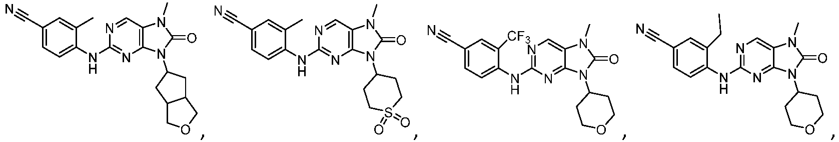

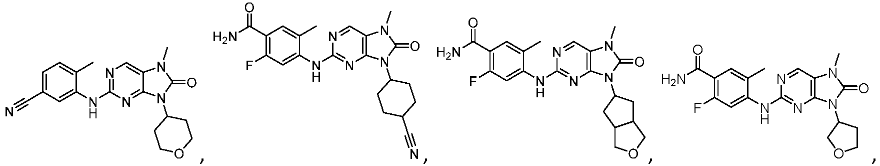

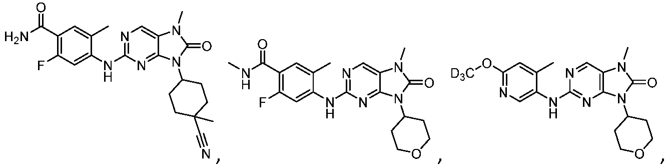

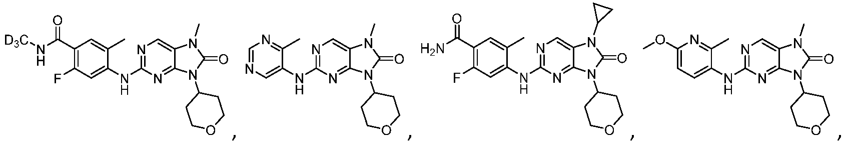

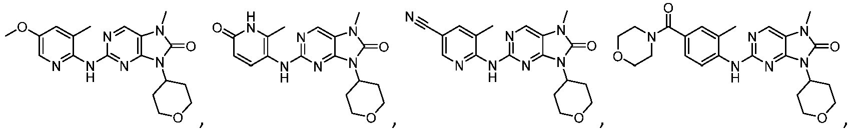

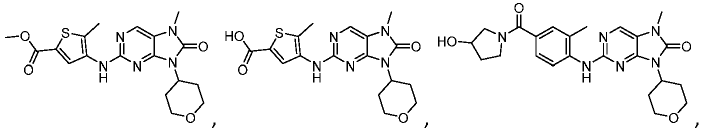

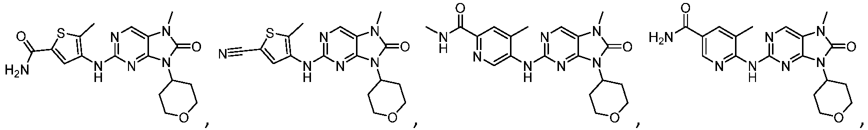

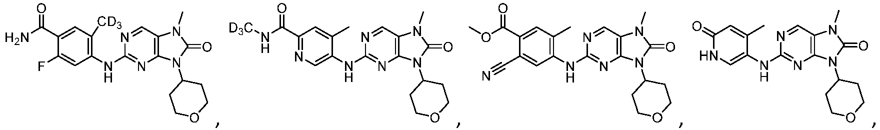

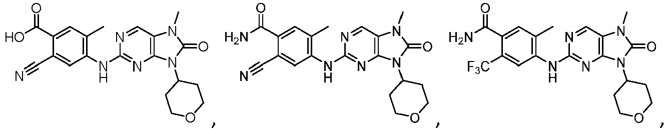

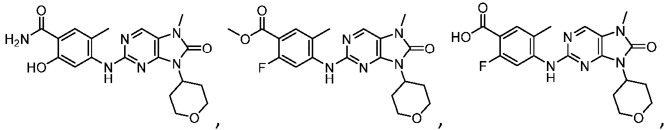

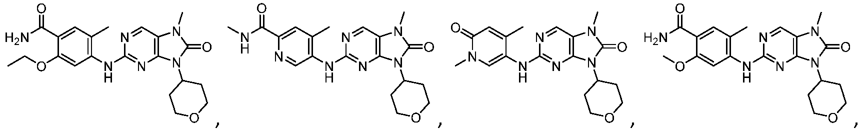

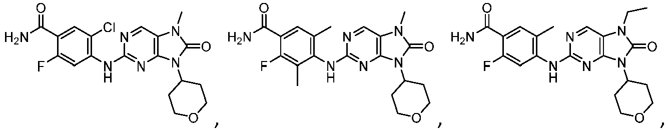

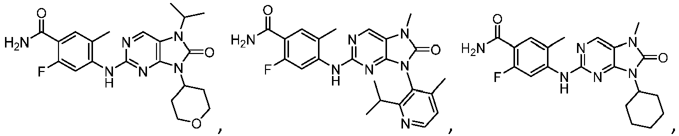

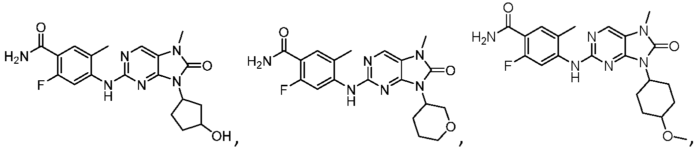

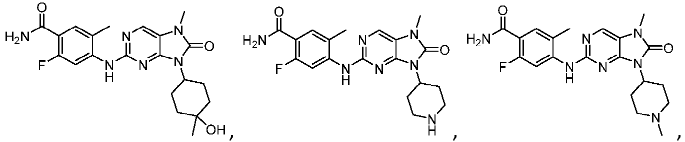

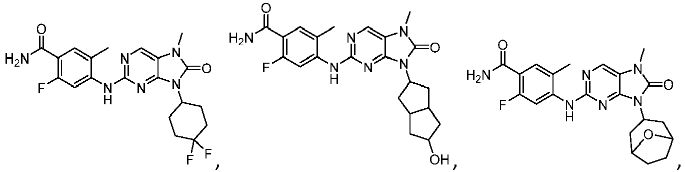

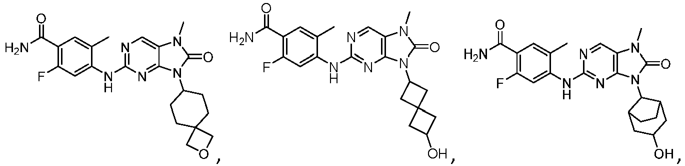

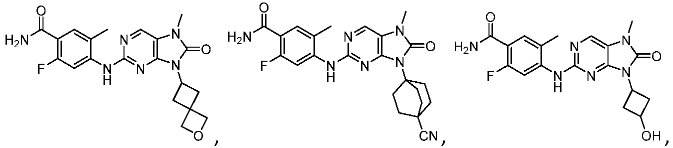

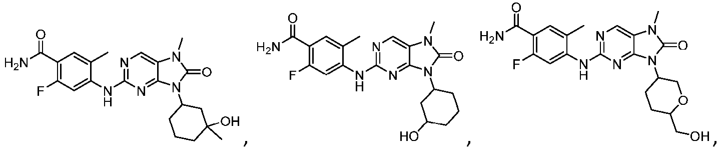

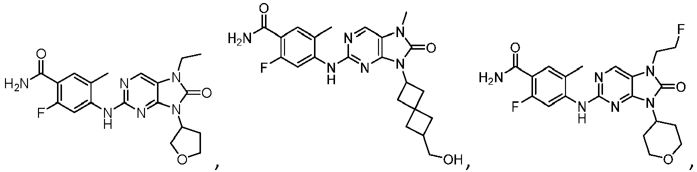

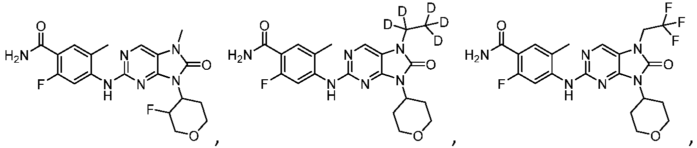

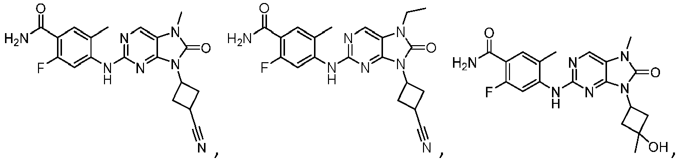

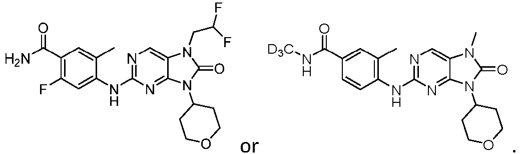

In one or more embodiments of the present application, the active ingredient exhibits obvious synergistical therapeutic effects when used in combination with Doxorubicin.

In one or more embodiments of the present application, the tumor is selected from a solid tumor.

In one or more embodiments of the present application, the daily dose of the active ingredient is 25-500 mg, preferably 50-400 mg, more preferably 100-300 mg, and the administration dose of the Doxorubicin or a pharmaceutically acceptable salt thereof is 0.02-10 mg/kg.

In one or more embodiments of the present application, the administration dose of the Doxorubicin or a pharmaceutically acceptable salt thereof is no more than 50 mg/m² body surface area of the subject.

### BRIEF DESCRIPTION OF THE DRAWINGS

FIG. 1 shows a compound A;
FIG. 2 shows a graph of the trend of the volume of tumor in an H1299 mouse tumor-bearing model; and
FIG. 3 shows a graph of the trend of body weight change of tumor-bearing nude mice in an H1299 mouse tumor-bearing model.

### DETAILED DESCRIPTION OF EMBODIMENTS

The implementation process and beneficial effects of the present invention are described in detail below through specific examples, which are intended to help readers better understand the essence and characteristics of the present invention, and are not intended to limit the scope of implementation of the present invention.

The present invention will be described in further detail below with reference to the accompanying drawings.

The compound A in the examples is compound 62 of WO 2021209055 and is prepared according to the preparation method therefor.

### Efficacy test in H1299 mouse tumor-bearing model

### 1. Experimental steps:

### 1.1 Cell culture

H1299 human lung cancer cells were purchased from ATCC. H1299 cells were subjected to adherent culture using a 15 cm culture dish under culture conditions of a DMEM medium supplemented with 10% fetal bovine serum and penicillin and streptomycin, and cultured in an incubator containing 5% CO₂ at 37°C. The cells in an exponential growth phase were digested with trypsin, and the cells were collected and counted, followed by inoculation.

### 1.2. Grouping and administration

BALB/c-nude mice were adapted to the laboratory environment and then inoculated subcutaneously with an H1299 cell suspension in the right flank at an inoculation amount of 7.6 × 10⁶ cells/mouse, with an inoculation volume of 0.2 mL (with added gel). When the tumors were grown to approximately 120 mm³, 24 animals were screened for grouping. The animals were divided into 3 groups using a zigzag-pattern grouping method based on the tumor volume, with 8 mice in each group. The day of grouping is defined as day 1 (PG-D1) of the experiment, and administration began on day 1. The grouping and administration regimen are shown in Table 1.

**Table 1 Administration regimen**

| Group No. | Group | Mode of administration | Number of animals |
|---|---|---|---|
| 1 | Blank (Vehicle) | - | 8 |
| 2 | Doxorubicin liposome | 5 mg/kg, QW, i.v. | 8 |
| 3 | Compound A + Doxorubicin liposome | 20 mg/kg, BID, i.g. + 5 mg/kg, QW, i.v | 8 |

| | | | |
|---|---|---|---|
| Notes: i.g. refers to intragastrical gavage; i.v. refers to tail vein administration; BID: dosing twice a day; and QW: dosing once a week. | | | |

### 2. Assay method

2.1 Tumor volume: during a drug treatment cycle, the long diameter (a) and short diameter (b) of tumor tissue were measured twice a week using an electronic vernier caliper to calculate the volume of the tumor (tumor volume = 0.5 × a × b²).
2.2 Body weight of mice: mice were weighed at least 2 times a week during a drug treatment cycle.

### 3. Data analysis

All data were input into SPSS 16.0 software, and the data were compared using One-Way ANOVA, with P < 0.05 considered a statistically significant difference.

### 4. Experimental results

### 4.1 Tumor volume results

**Table 2-1. Mean tumor volume of tumor-bearing nude mice (mm³)**

| Group | PG-DO | PG-D4 | PG-D8 | PG-D11 | PG-D15 |
|---|---|---|---|---|---|
| Blank (Vehicle) | 124 ± 12 | 244 ± 35 | 585 ± 70 | 889 ± 82 | 1443 ± 139 |
| Doxorubicin liposome | 124 ± 12 | 230 ± 34 | 474 ± 54 | 677 ± 67 | 850 ± 105 |
| Compound A + Doxorubicin liposome | 123 ± 11 | 221 ± 18 | 251 ± 21***^{#} | 281 ± 29***^{##} | 262 ± 36**^{##} |

**Table 2-2. Mean tumor volume of tumor-bearing nude mice (mm³)**

| Group | PG-D18 | PG-D22 | PG-D25 | PG-D29 |
|---|---|---|---|---|
| Blank (Vehicle) | 1924 ± 172 | 2802 ± 250 | 3525 ± 303 | 4573 ± 286 |
| Doxorubicin liposome | 1022 ± 174* | 1177 ± 234** | 1294 ± 297** | 1421 ± 361*** |
| Compound A + Doxorubicin liposome | 253 ± 50***^{##} | 207 ± 61***^{##} | 161 ± 54***^{#} | 142 ± 54***^{##} |

| | | | | |
|---|---|---|---|---|
| Notes: PG-D1 is the time of first administration; P value refers to comparison with Vehicle: **P* < 0.05, ***P <* 0.01, and ***P < 0.001; and P value refers to Compound A + Doxorubicin liposome as compared to Doxorubicin liposome: #P < 0.05, and ##P < 0.01. | | | | |

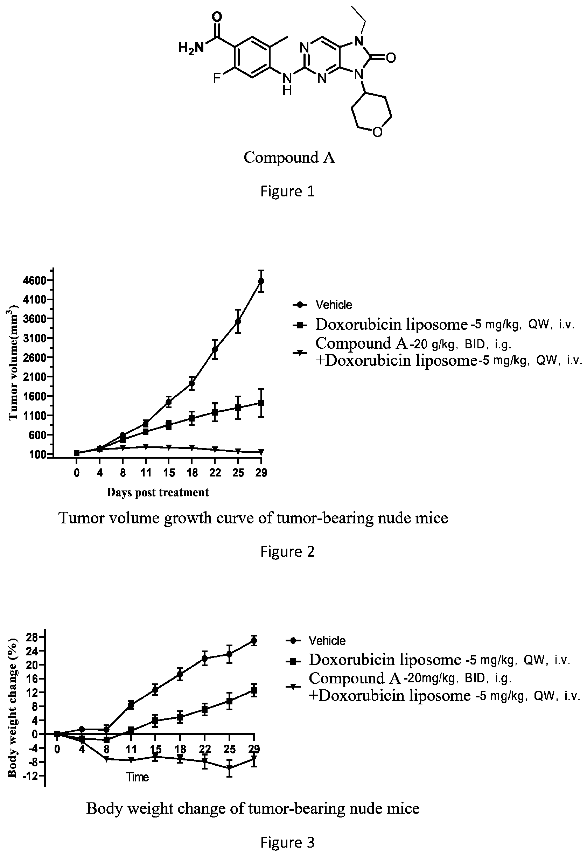

As shown in Table 2-1, Table 2-2, and FIG. 2, by the end of administration (PG-D29), the tumor volume in the group administered with compound A in combination with Doxorubicin liposome is significantly smaller than that in the blank group and the Doxorubicin liposome group (P < 0.01), indicating that compound A can significantly enhance the anti-tumor effect of the Doxorubicin liposome.

### 4.2 Body weight results

**Table 3-1. Body weight of tumor-bearing nude mice (g)**

| Group | PG-D0 | PG-D4 | PG-D8 | PG-D11 | PG-D15 |
|---|---|---|---|---|---|
| Blank (Vehicle) | 18.16 ± 0.16 | 18.41 ± 0.24 | 18.40 ± 0.32 | 19.70 ± 0.34 | 20.50 ± 0.41 |
| Doxorubicin liposome | 18.63 ± 0.38 | 18.36 ± 0.35 | 18.33 ± 0.47 | 18.83 ± 0.53 | 19.35 ± 0.58 |
| Compound A + Doxorubicin liposome | 18.58 ± 0.29 | 18.19 ± 0.32 | 17.24 ± 0.24 | 17.18 ± 0.37 | 17.38 ± 0.45 |

**Table 3-2. Body weight of tumor-bearing nude mice (g)**

| Group | PG-D18 | PG-D22 | PG-D25 | PG-D29 |
|---|---|---|---|---|
| Blank (Vehicle) | 21.30 ± 0.44 | 22.13 ± 0.45 | 22.36 ± 0.61 | 23.06 ± 0.38 |
| Doxorubicin liposome | 19.54 ± 0.51 | 19.95 ± 0.53 | 20.40 ± 0.58 | 20.98 ± 0.50 |
| Compound A + Doxorubicin liposome | 17.25 ± 0.38 | 17.10 ± 0.49 | 16.74 ± 0.49 | 17.24 ± 0.44 |

As shown in Table 3-1, Table 3-2, and FIG. 3, by the end of administration (PG-D29), the animals in the group administered with compound A in combination with Doxorubicin liposome show a slight decrease in body weight compared to the initial weight thereof, but show overall good tolerance.

Specific embodiments are described in detail in the description of the present invention. A person skilled in the art should recognize that the embodiments described above are exemplary and cannot be construed as limiting the present invention. Additionally, a person skilled in the art can make several improvements and modifications to the present invention without departing from the principle of the present invention, and the technical solutions obtained based on these improvements and modifications also fall within the scope of protection of the claims of the present invention.

## Claims

1. Use of a compound represented by formula (I), or a stereoisomer, solvate, prodrug, metabolite, pharmaceutically acceptable salt or co-crystal thereof as an active ingredient for the preparation of an anti-tumor drug in combination with Doxorubicin: 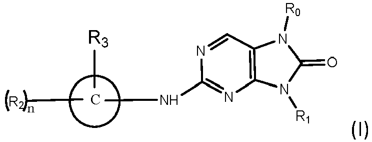 wherein 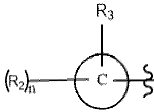 is 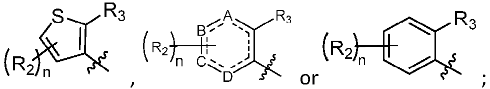
-̅ -̅ -̅ is a single bond or double bond;
in 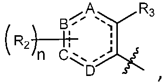 A, B, C, D are each independently C or N, and at least one of A, B, C and D is N;
R₀ is H, C₁₋₆ alkyl or cyclopropyl, wherein the C₁₋₆ alkyl is optionally further substituted with one or more substituents selected from halogen and deuterium;
R₁ is 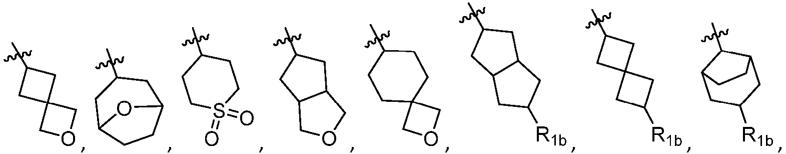 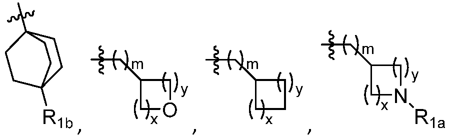 or pyridyl, and R₁ is optionally further substituted with 1 or 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R₂ is H, cyano, =O, carboxyl, -C(=O)NR₂ₐR_{2b}, C₁₋₆ alkoxy, C₁₋₆ alkyl, halogen, -S(=O)₂R₂ₐ or - C(=O)OC₁₋₆ alkyl, wherein the C₁₋₆ alkyl, -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1, 2 or 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 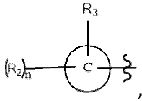 R₀, R₂, and R₃ simultaneously satisfy the following conditions, R₁ is not 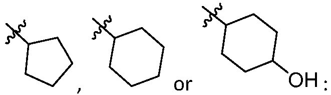 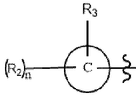 is 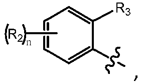 n is 1, R₀ is H or methyl, R₂ is methoxy or -S(=O)₂Me, and R₃ is methyl.

2. The use according to claim 1, wherein the active ingredient is the compound of formula (I), or a stereoisomer, solvate, prodrug, metabolite, pharmaceutically acceptable salt or co-crystal thereof, wherein
R₁ is 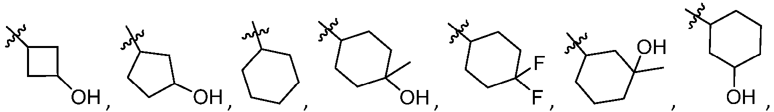 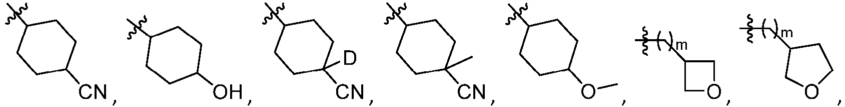 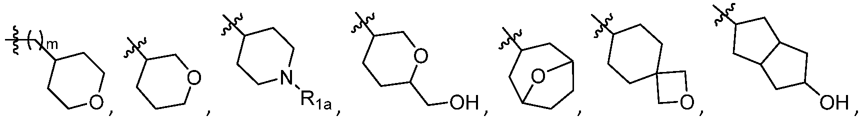 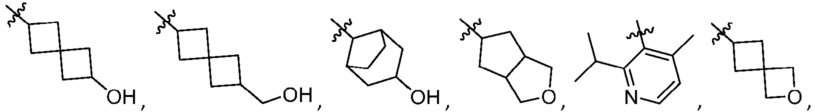 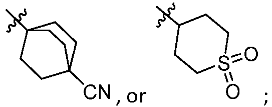
R₁ₐ is H or C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 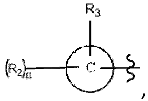 R₀, R₂, and R₃ simultaneously satisfy the following conditions, R₁ is not 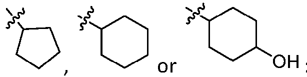 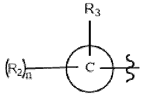 is 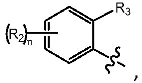 n is 1, R₀ is H or methyl, R₂ is methoxy or -S(=O)₂Me, and R₃ is methyl.

3. The use according to claim 1, wherein the active ingredient is the compound of formula (I), or a stereoisomer, solvate, prodrug, metabolite, pharmaceutically acceptable salt or co-crystal thereof, wherein 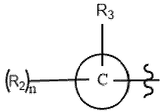
is 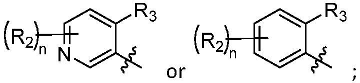 R₀ is H, C₁₋₄ alkyl or cyclopropyl, wherein the C₁₋₄ alkyl is optionally further substituted with one or more substituents selected from halogen and D;
R₁ is 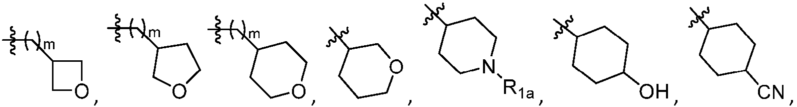 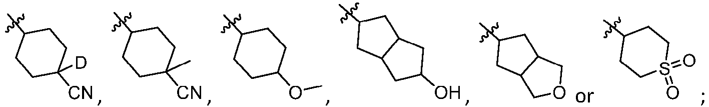
R₁ₐ is H, C₁₋₆ alkyl or -C(=O)C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D and halogen;
alternatively, R₂ₐ and R_{2b} together with the atoms to which they are attached form a 5- to 6-membered heterocyclyl, which contains 1 to 3 heteroatoms selected from N, O and S and is also optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 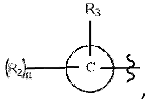 R₀, R₂, and R₃ simultaneously satisfy the following conditions, R₁ is not 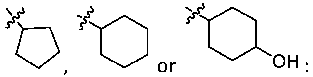
when 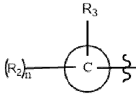 is 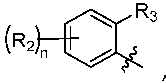 n is 1, R₀ is H or methyl, R₂ is methoxy or -S(=O)₂Me, and R₃ is methyl.

4. The use according to claim 1, wherein the active ingredient is selected from a compound of formula (II), or a stereoisomer, solvate, prodrug, metabolite, pharmaceutically acceptable salt or co-crystal thereof: 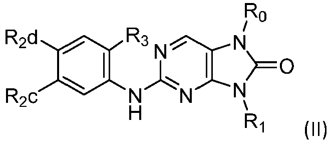 wherein
R₀ is H, C₁₋₆ alkyl or cyclopropyl, wherein the C₁₋₆ alkyl is optionally further substituted with one or more substituents selected from halogen and deuterium;
R₁ is 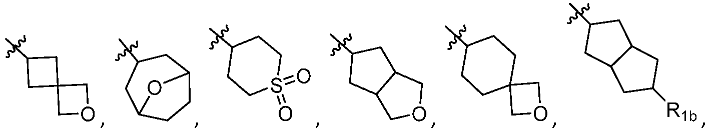 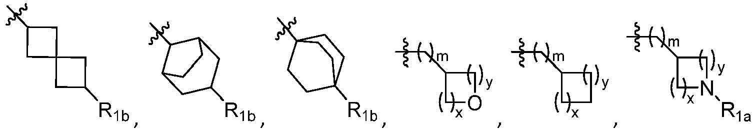 or pyridyl,
and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R_{2c} is H, cyano, halogen or C₁₋₆ alkoxy;
R_{2d} is H, cyano, carboxyl, -C(=O)NR₂ₐR_{2b}, C₁₋₆ alkyl, halogen, -S(=O)₂R₂ₐ or -C(=O)OC₁₋₆ alkyl, wherein the C₁₋₆ alkyl and -C(=O)OC₁₋₆ alkyl is optionally substituted with one or more substituents selected from halogen and deuterium;
R₂ₐ and R_{2b} are H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 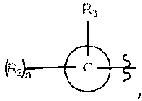 R₀, R₂, and R₃ simultaneously satisfy the following conditions, R₁ is not 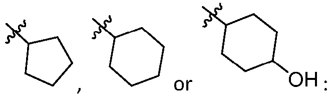 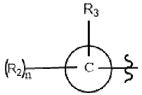 is selected from 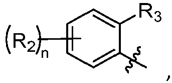 n is 1, R₀ is H or methyl, R₂ is methoxy or - S(=O)₂Me, and R₃ is methyl.

5. The use according to claim 4, wherein the active ingredient is selected from a compound of formula (III), or a stereoisomer, solvate, prodrug, metabolite, pharmaceutically acceptable salt or co-crystal thereof: 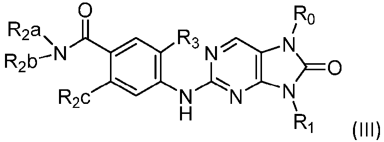 wherein
R₀ is H, C₁₋₆ alkyl or cyclopropyl, wherein the C₁₋₆ alkyl is optionally further substituted with one or more substituents selected from halogen and deuterium;
R₁ is 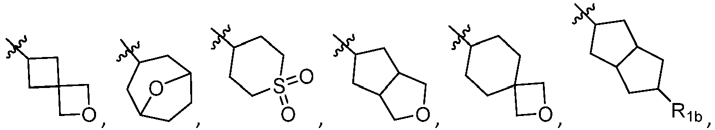 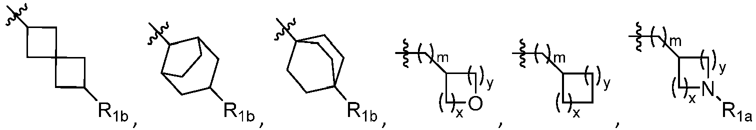 or pyridyl,
and R₁ is optionally further substituted with 1 to 2 substituents selected from D, halogen, cyano, hydroxyl, C₁₋₆ alkyl and C₁₋₆ alkoxy;
R_{1b} is H, OH, cyano, or hydroxyl substituted C₁₋₆ alkyl;
R₂ₐ and R_{2b} are each independently H, C₁₋₆ alkyl, or 3- to 5-membered cycloalkyl, wherein the C₁₋₆ alkyl is optionally further substituted with one or more substituents selected from D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R_{2c} is H, cyano, halogen or C₁₋₆ alkoxy, wherein the C₁₋₆ alkoxy is optionally substituted with one or more deuterium;
R₃ is halogen or C₁₋₆ alkyl, wherein the C₁₋₆ alkyl is optionally further substituted with 1 to 3 substituents selected from D or halogen;
m is 0 or 1;
n is 0, 1 or 2; and
x and y are each independently 1, 2 or 3.

6. The use according to claim 1, wherein the active ingredient is selected from a compound of formula (IV), or a stereoisomer, solvate, prodrug, metabolite, pharmaceutically acceptable salt or co-crystal thereof: 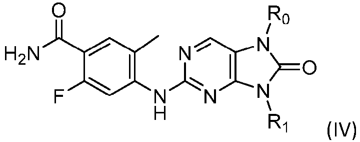 wherein
R₀ is H, C₁₋₆ alkyl or cyclopropyl, wherein the C₁₋₆ alkyl is optionally further substituted with one or more substituents selected from halogen and deuterium; and
R₁ is -(CH)ₘ-4- to 7-membered carbocyclyl, -(CH)ₘ-4- to 7-membered heterocyclyl, -(CH)ₘ-8- to 12-membered bridged ring, -(CH)ₘ-7- to 12-membered spiro ring, wherein the -(CH)ₘ-4- to 7-membered carbocyclyl, -(CH)ₘ-4- to 7-membered heterocyclyl, -(CH)ₘ-8- to 12-membered bridged ring, or -(CH)ₘ-7- to 12-membered spiro ring is optionally further substituted with one or more substituents selected from hydroxy, cyano, halogen, =O, C₁₋₆ alkyl, C₁₋₆ alkoxy, and hydroxy substituted C₁₋₆ alkyl.

7. The use according to claim 6, wherein the active ingredient is selected from the compound of formula (IV), or a stereoisomer, solvate, prodrug, metabolite, pharmaceutically acceptable salt or co-crystal thereof, wherein
R₀ is H, C₁₋₆ alkyl or cyclopropyl, wherein the C₁₋₆ alkyl is optionally further substituted with one or more substituents selected from halogen and deuterium;
R₁ is 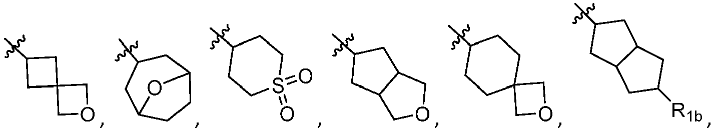 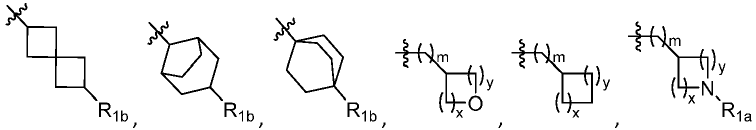 or pyridyl,
and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
m is 0 or 1; and
x and y are each independently 1, 2 or 3.

8. The use according to claim 7, wherein the active ingredient is the compound of formula (IV), or a stereoisomer, solvate, prodrug, metabolite, pharmaceutically acceptable salt or co-crystal thereof, wherein
R₀ is C₁₋₄ alkyl, wherein the C₁₋₄ alkyl is optionally further substituted with one or more substituents selected from halogen and deuterium; and
R₁ is

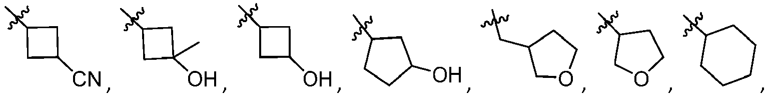

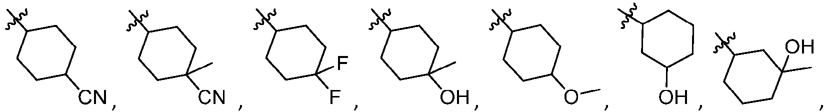

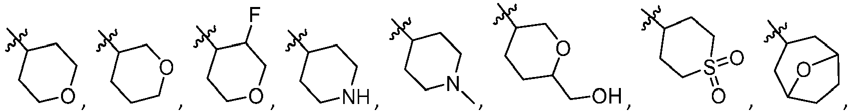

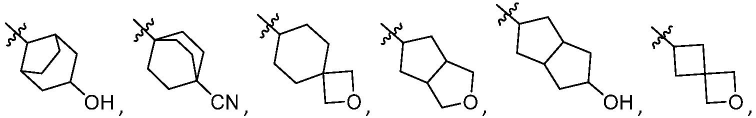

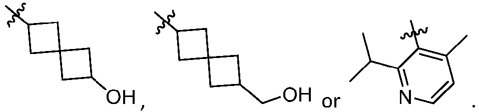

9. The use according to any one of claims 1-8, wherein the active ingredient is selected from: 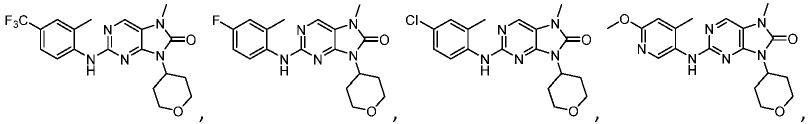 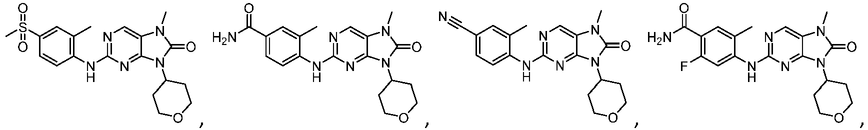 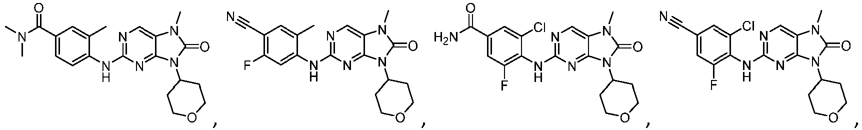 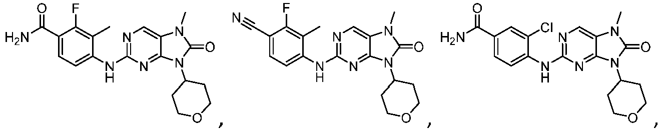 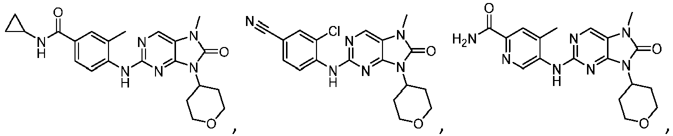 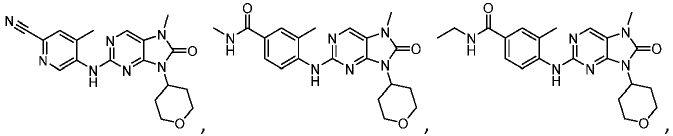 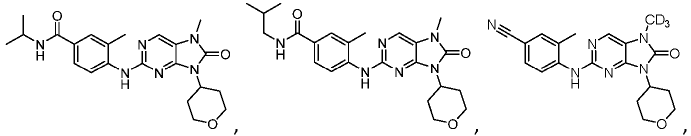 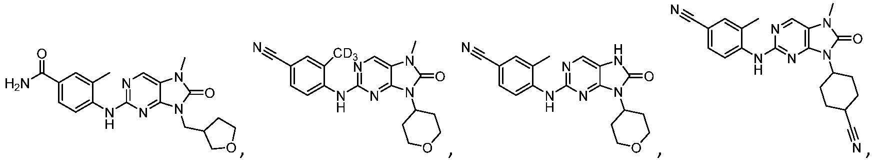 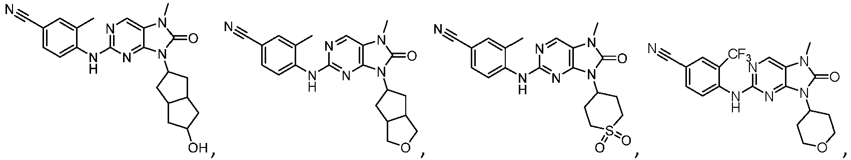 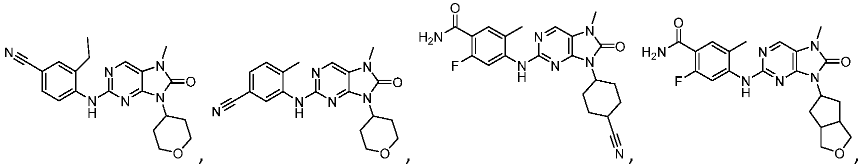 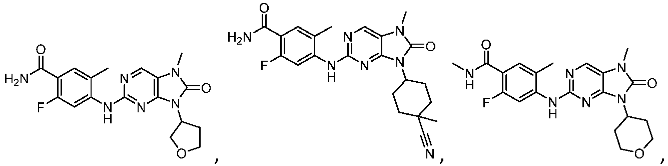 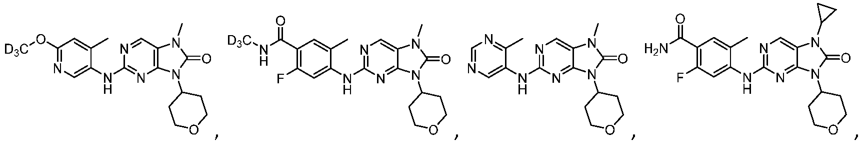 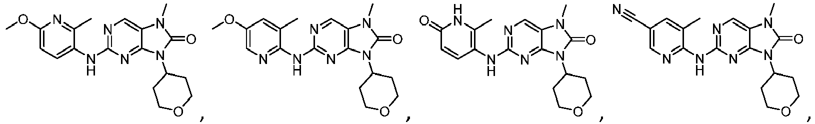 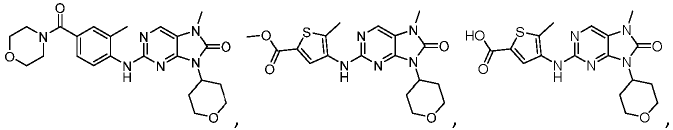 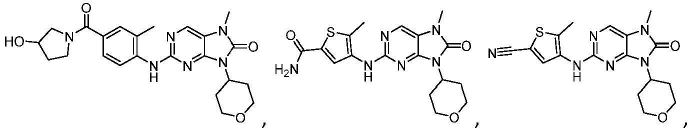 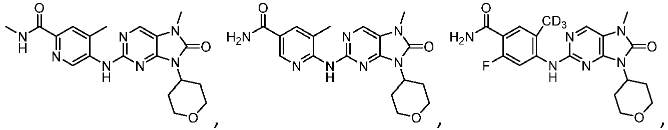 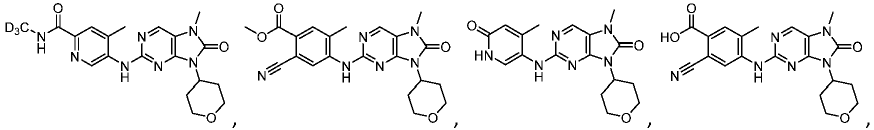 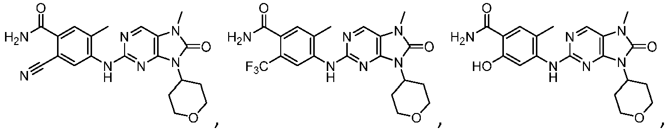 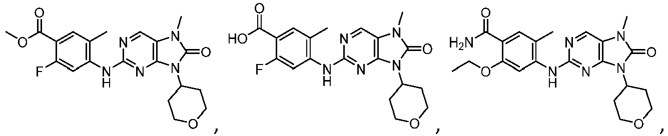 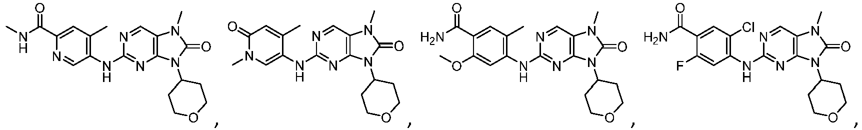 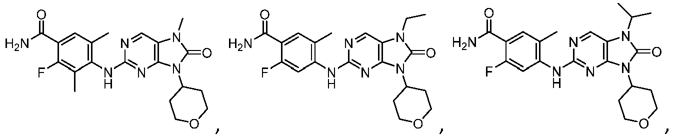 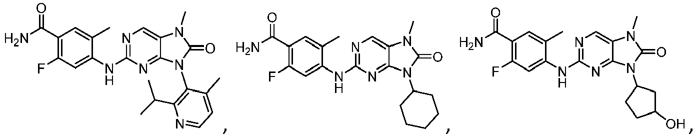 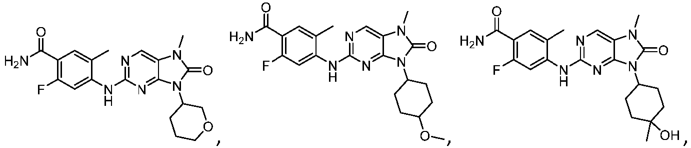 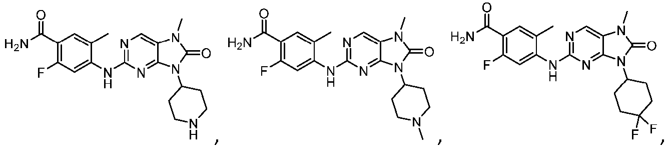 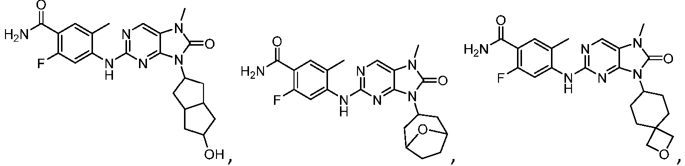 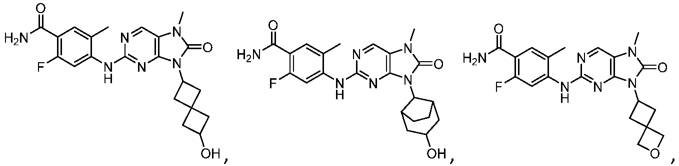 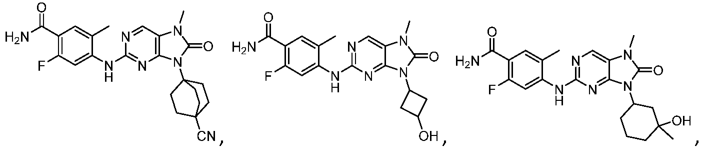 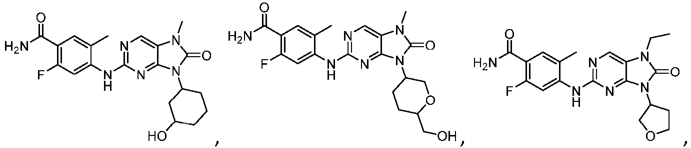 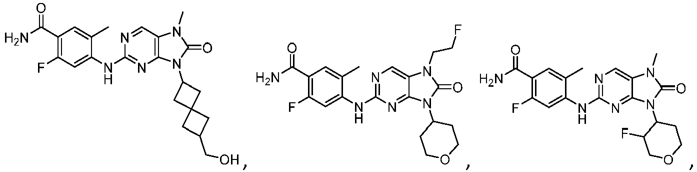 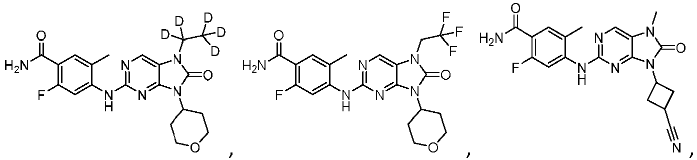 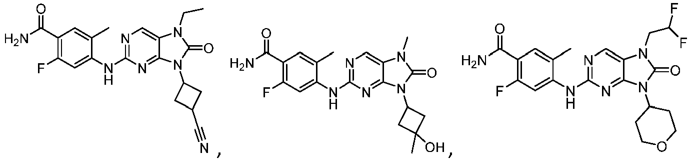 or

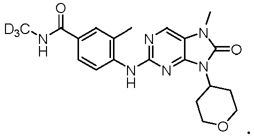

10. The use according to any one of claims 1 to 9, wherein the tumor is selected from a solid tumor.

11. The use according to any one of claims 1 to 9, wherein the daily dose of the active ingredient is 25-500 mg, preferably 50-400 mg, more preferably 100-300 mg, and the administration dose of the Doxorubicin or a pharmaceutically acceptable salt thereof is 0.02-10 mg/kg.

12. The use according to claim 11, wherein the administration dose of the Doxorubicin or a pharmaceutically acceptable salt thereof is no more than 50 mg/m² body surface area of a subject.
